# EUROPEAN PATENT APPLICATION

(11) **EP 2 047 853 A1**
(43) Date of publication of application: **15.04.2009**
(21) Application number: 06775552.0
(22) Date of filing: 31.08.2006
(51) Int. Cl.: A61K 31/56, A61P 9/10, A61P 25/00

(54) **THE APPLICATION OF MARINE STEROID IN PREPARING THE MEDICINE OF TREATING NEURONS DAMAGING**

(30) Priority: 03.08.2006 CN 200610036926
(71) Applicant: Sun Yat-Sen University, Guangzhou, Guangdong 510089 (CN)
(72) Inventor: YAN, Guangmei, Guangdong 510089 (CN); QIU, Pengxin, Guangdong 510089 (CN); HUANG, Yijun, Guangdong 510089 (CN); YIN, Wei, Guangdong 510089 (CN); SU, Xingwen, Guangdong 510089 (CN); LIU, Ailing, Guangdong 510089 (CN); SANG, Hanfei, Guangdong 510089 (CN); CAI, Xiang, Guangdong 510089 (CN)
(74) Representative: Kramer - Barske - Schmidtchen
(86) International application number: PCT/CN2006/002235
(87) International publication number: WO 2008/017216

(57) **Abstract**

The application of marine steroid, i.e. 24-methylene-cholest-3β,5α,6β,19-tetrol, in preparing the medicine of treating neurons damaging is provided in the present invention. The keto-sterols compounds marine steroid YC-1 extracted from Nephthea albida has the action of neuronal protection, and no toxic reaction under the effective protective dosage.

## Description

### FIELD OF THE INVENTION

This invention relates to the application of neuron related steroids, concretely the use of marine steroid YC-1 (24-methylene-cholest-3β,5α,6β,19-tetrol) in preparing medicines for treating neuron damages.

### BACKGROUND OF THE INVENTION

Cerebrovascular disease happens frequently and has become one of the three major diseases endangering human's health. Among annual death number caused by diseases, that caused by cerebrovascular ranks 2nd in Japan, 3rd in the US and has always been No.1 in China recent years. Cerebrovascular disease can be basically considered as a senile disease. Its incidence increases with age. For people above 55, the incidence doubles in every decade. As increasing of living standard, prolonging of longevity and intensifying of population aging, the incidence of cerebrovascular disease is constantly increasing. At the same time, sufferers of cerebrovascular disease are becoming younger and younger due to change of life style. Among cerebrovascular diseases, about 60-80% is caused by ischemia (abbr. cerebral ischemia). The main reason for cerebral ischemia is cerebral arteriosclerosis. The mechanism of cerebral arteriosclerosis is as follows: systemic arteriosclerosis usually happens first, followed by general atherosclerosis, angiostenosis and small vessel occlusion, which reduces the blood supply for brain parenchyma, even causes cerebral thrombosis, complete vessel occlusion and extensive acute infarct of relevant blood supplying brain tissues, which is called stroke or apoplexy, this will finally lead to brain dysfunction or even permanent function losing. The high incidence, morbidity, mortality, recurrence rate or cerebral ischemia and symptoms caused by them, like hemiplegia, aphasia, dementia, have become heavy burdens of modem families.

So far there are two kinds of major treatment for damages caused by cerebral ischemia: to improve and recover blood supply of the damaged brain tissue asap or protect the tissue from further injury of toxic metabolites. The aim of recanalization of the occlusive vessels asap is to supple blood for involved brain tissue timely, block the chain reaction of chemicals killing neurons, enhance the tolerance of brain tissue, and salvage functions of those tissue in penumbral area before irreversible damage happens. While protection of neurons can be achieved by blocking various harmful pathological process caused by ischemia, preventing brain damages induced by localized ischemia, reducing brain cell death and promoting functional recovery. If both of them are applied, they may cause synergistic effect, produce more pertinent and scientific therapy and improve the curative effect.

Means to improve and recover blood supply for ischemic brain tissue include thrombolysis, anticoagulation, and anti-platelet aggregation, et al.

### 1. Thrombolysis

Thrombolysis especially those of ultra-early and high-dose has become the first choice for ischemic brain damage since 1980s. Comparing with the placebo group, thrombolysis significantly increased the incidence of intracranial hemorrhage and other death. So the risk of thrombolysis is high and doctors have to master the indications and administration time, especially for areas of long-time ischemia, such as ischemic core and those of vulnerabal, for easy occurrence of reperfusion injury, postinfarction hemorrhage and severe brain edema.

### 2. Anticoagulation

It is very important to apply anticoagulation asap for although anticoagulation can prevent the conversion of prothrombin to thrombin and fibrinogen to fibrin together with platelet aggregation, it has no direct effect on existing thrombosis. However some scholars hold different ideas. They think immediate anticoagulation (like heparin, low mocular heparin, heparin factors, or special thrombin inhibitors) at the moment of thrombosis didn't show long- or short-term improving effect. Although immediate therapy may reduce thrombosis in deep venous and pulmonary artery, this effect was counteracted by the risk of intra- and extracranial hemorrhage.

### 3.Anti-platelet aggregation

With function of anti-platelet aggregation, aspirin has been widely used on ischemic brain damage, but the dosage has not been unified during these years. Laboratory research proved that about 30-50mg/d of aspirin is enough to do the job. So clinically low dose aspirin has been used for treatment and prevention of ischemic cerebrovascular disease. These years new anti-platelet medicine-ticlopidine has been used on ischemic cerebrovascular disease and its clinical effect is recognized better than aspirin.

### 4.Hemodilution

Hemodilution therapy functions through lowering packed cell volume , reducing blood viscosity and increasing regional cerebral blood flow. According to literature reports, early normovolemic hemodilution and adhesive exudates with long half-life (like synthesized protein Pentastarch who can carry oxygen or albumin) etc are expected to show better effects. Selective blood component dilution is a new therapy based on hemodilution and ultraviolet blood irradiation therapy used worldwide. It changes or abandons plasma or abandons blood cells according to different kinds of blood rheology of ischemic brain damaged patients.

### 5.Vasodilative medicine

Common used vasodilative medicines are cinnarizine, papaverine, calcium bicarbonate and calcium antagonist nimodipine, et al. However some people don't intent to use vasodilative medicines for they think application of them at the acute stage of ischemic cerebrovascular can cause reperfusion injury easily and aggravate brain tissue damages of penumbral area.

On the other hand, it has recently become a research hotspot of cerebral ischemic therapy to protect the ischemic brain tissue and neurons. Many neuroprotective agents are in clinical development. Main action pathways of neuroprotective agents for acute ischemic brain damage include: preventing influx of Ca²⁺, clearing the free radicals, using excitatory amino acid receptor antagonist, neurotrophic factor, and γ-aminobutyric acid receptor agonist et al. More attention has been focused on the following pathways:

### 1. Ca²⁺ channel blocker

The earliest and most extensively studied Ca²⁺ channel blocker is dihydropyridine type medicines. Let's take nimodipine as an example. As voltage-sensitive calcium channel antagonist, nimodipine is lipophilic and easy to pass blood-brain barriers. Among randomized controlled study with 3719 patients, no significant effect was seen with 120mg oral administration; The risk of bad result decreased 38% for patients receiving treatment within 12hrs from onset. The effect of using nimodipine within 6hrs is still in process of clinical III research in Netherlands. Small sample research of magnesium shows good patient tolerance; Most patients treated showed neural functional improvement and less readmission within 6 months. Study from a group of 60 patients showed that after regular magnesium sulfate treatment, the mortality and morbidity rate for the treated group is 30% and the number is 40% for the placebo group and study of the exact effect of this medicine is still in process.

### 2.Free radical scavenger

Many free radicals are produced in acute cerebral ischemia and this will damage cell membrane and neurons. The free radicals also cause vasospasm and intravascular coagulation in the penumbral area, widen the infracted area and exacerbate the brain tissue damage. So it is crucial to scavenge the free radicals. Vitamin E, vitamin C, superoxide dismutase(SOD), hormone and mannitol etc are commonly used free radical scavenger and they can protect ischemic brain cells.

### 3.Glutamic acid release inhibitor

Glutamic acid release inhibitors cure acute ischemic brain damage through inhibiting the synthesis and release of presynaptic glutamic acid. Animal experiments showed glutamic acid release inhibitors have obvious brain protection function but its clinical application is to be verified.

Researchers worldwide have always devoted to solve the problem of post-ischemia brain cell protection. For the pathological mechanism of neural tissue and cell damage induced by cerebral ischemia involves complex time-space cascade and reciprocal reactions, any interferefering therapeutic test merely aiming at a single step or molecular mechanism is almost doomed to bring little effect. That is why most clinical tests are disappointing so far in spite of the promising therapeutic methods verified on animal models and constant effort for developing anti-cerebral ischemia chemicals. Maybe it is because of the neglect of the multistage, multi-center mechanism and multi-direction character of the cell damage process derived from cerebral ischemia.

On the whole, although human been has deeply understood the mechanism and pathological changes of cerebral ischemia, there is still a great need of relevant effective therapeutic means. So far only thrombolysis and recanalization are mainly used for cerebral ischemia. However these treatments only recovered the blood supply, there are still risk of hemorrhage and other limitations in application, and they are almost helpless for post-ischemic neuron damages especially reperfusion injury and delayed death. Although general efficacy is not ideal, the yearly expenditure on treating apoplexy reaches hundred billion RMB, and this doesn't include the huge cost for the long term brain damage recovery and neural functional maintain that may occur among the patient's whole life. Thus it is extraordinary important to find medicines interfering cerebral ischemia's development in multiple steps and mechanisms. These medicines will have a profound influence on therapy of cerebral ischemia, greatly reduce the spiritual and economic burden of the society, family and individuals, take a place in the great potential medicine market, and bring remarkable social and economic benefits.

Some pharmacologists predict promising effective anti-cerebral ischemia medicines would arise from chemicals that can bind multiple targets. Among the numerous research objects, neuroactive steroids are gaining increasing attention for their widely effect on nervous system.

Neuroactive steroids(NASs) is the general designation of all natural or synthesized neuroactive steroid hormones. NASs have the same typical action mode as general steroid hormones which is regulating various receptors and proteins' gene expression in neuron and glial cells, and this is called genomic effect. Genomic effect includes DNA transcription, translation and protein modification and it functions slowly. NASs can also fast modulate activity of GABA receptor, Glu receptor, acetylcholine receptor and intracellular Sigmal receptor, directly affect central activities (like emotion, neuronal plasticity and excitability, synaptic transmission, learning and memory, et al) in non-genomic way. Recent research showed some NASs' have obvious modulation on neuronal damage, death, and variant central nervous system diseases. For example, kainic acid can cause hippocampus CA1 and CA3 neuron loss while allopregnanolone (AL) can relieve the loss. AL also showed such protection in irreversible neurotoxicity cell model cultured in vitro (low oxygen and Glu). Though dehydroepiandrosterone (DHEA) can decrease the neurotoxicity of Glu analogues and corticosteroid, its concentration in vivo decreases with age and this is considered to correlate with neurodegenerative change accompanying aging. DHEA also showed such protection in reversible rabbit spinal cord ischemia model. Certain amount of DHEA can significantly increase in vitro rat embryo cortical center anoxia neuronal viability. Estrogen is known as the most powerful neuro-protective NAS. Consistent with Parkinson's Disease's (PD) gender differences, estrogen has fine protection on nigral dopaminergic neurotoxicity. Postmenopausal women stop secreting estrogen, this will cause increase of amyloid precursor protein in brain and sedimentary of Aβ, while Aβ's level decreases with administration of estrogen. This suggests the internal connection between estrogen and Alzheimer's Disease (AD). Estrogen can not only decrease neuronal death caused by ischemia, apoplexy and many other factors, but also postpone kainic acid induced epilepsy and reduce epilepsy caused hippocampal damage. Estrogen's obvious neuronal protection in several toxicity models suggests its various functions on receptor activation, antioxidation, antagonizing biological toxicity, regulating signal transduction pathways and gene expression, et al. Some researchers showed that estradiol and testosterone have similar neurotrophic function and they can promote regeneration after central nervous system damage. Like calcium channel antagonists, estradiol can decrease cerebral ischemic reperfusion injury, decrease and postpone animal's epilepsy attack. Estradiol and plant estrogen can decrease the central nerve system cholinergic neuron degeration in castrated rats, reduce the loss of basal forebrain cholinergic neuron and hippocampal cholinergic nerve fibers, and improve animal's cognitive ability.

### DESCRIPTION OF THE INVENTION

Marine steroid YC-1 (24-methylene-cholest-3β,5α,6β,19-tetrol) is an keto-sterol with uncommon side chain. Some documents show YC-1 can not only improve learning and memory ability, but also inhibit Glu induced cerebellar granule neuron excitotoxin death in a non-genomic dose dependent and non-NMDA receptor mediated way. YC-1's dose-dependent inhibition on low potassium induced cerebellar granule neuron apoptosis suggests YC-1 plays its role through inhibiting c-Jun's gene expression and reducing c-Jun's phosphorylation, and PI3K/AKT pathway may be involved in the process. YC-1 can also dose-dependently inhibit cerebellar granule neuron apoptosis induced by hypoxia. In whole animal experiment, both behavioral index and tissue's activity assay indicated YC-1 has obvious preventive and therapeutic effect on not only rat MCAO induced focal cerebral ischemia injury but also rabbit spinal cord ischemia injury. YC-1 and its analogue soft coral cholestene can indirectly show its antioxidant defense through repressing glutathione(gsh)'s exhaustion etc, and show its anti-inflammatory function through stabilizing membrane and decreasing inflammatory factor PEG2s' release. Thus YC-1 may be an effective anti-ischemic brain damage chemical aiming at multiple targets and through various mechanisms and a potential clinical medicine anti-ischemic brain damage. Excitotoxin, oxidative stress, inflammatory response , neuronal apoptosis and necrosis play an important role in development of neurodegenerative diseases like PD(Parkinson's Disease) and AD (Alzheimer's Disease). Thus YC-1 is a greatly promising candidate of medicines treating neurodegenerative diseases.

The keto-sterols compounds marine steroid YC-1 extracted from Nephthea albida is protective to various neuron damages and showed no toxic reaction under effective protective dosage. The yield of YC-1 extracted from natural resources is limited and is not suitable for mass production. However synthesis can solve the problem. Starting with cheap hyodeoxycholic acid, YC-1 can be synthesized through 14 steps of organic reactions; Then characterize the chemical with data like IR, 1H NMR, 13C NMR, MS and EA got from techniques like IR, NMR, MS and EA etc.

This invention lays a firm foundation for optimizing and perfecting YC-1's structure and efficiency, finishing pre-clinical and clinical trials, and developing YC-1 to a new neuron protective medicine.

### BRIEF DESCRIPTION OF THE DRAWINGS

Fig.1 represents the synthetic route of YC-1 (24-methylene-cholest-3β,5α,6β, 19-tetrol).

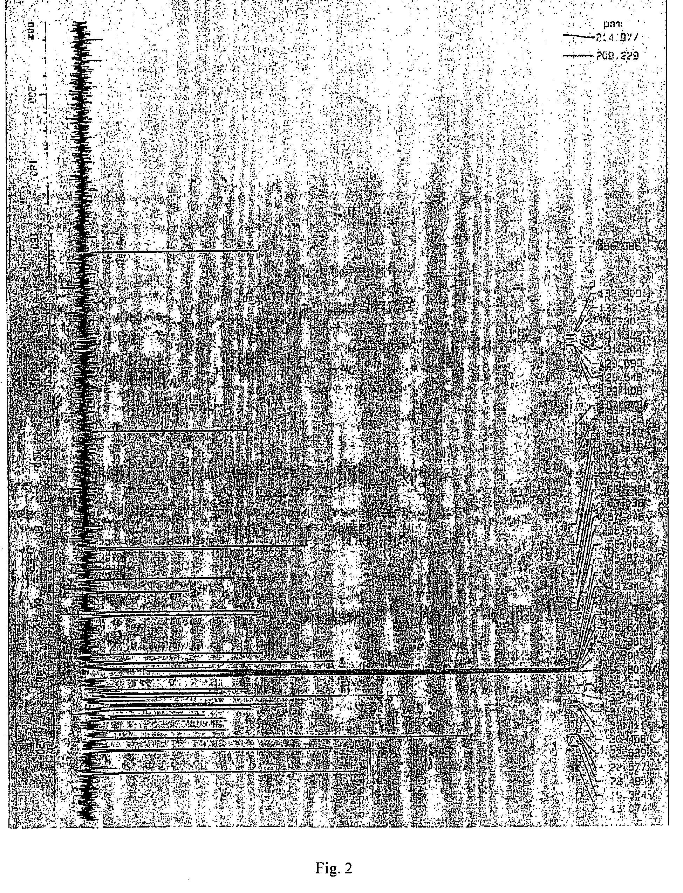

Fig.2 represents YC-1's c-nmr spectra.

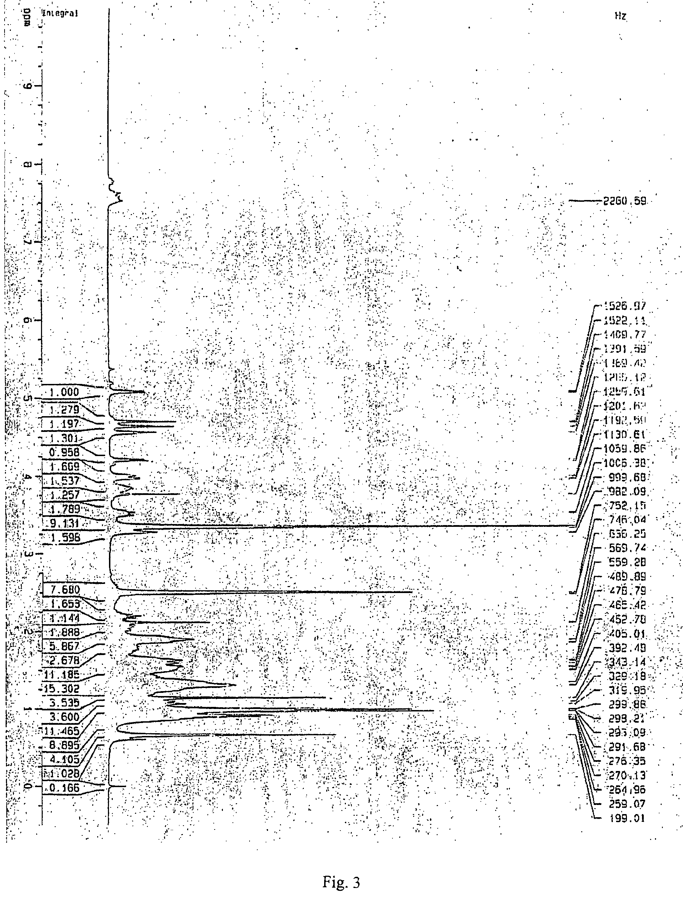

Fig.3 represents YC-1's 1h nmr spectra.

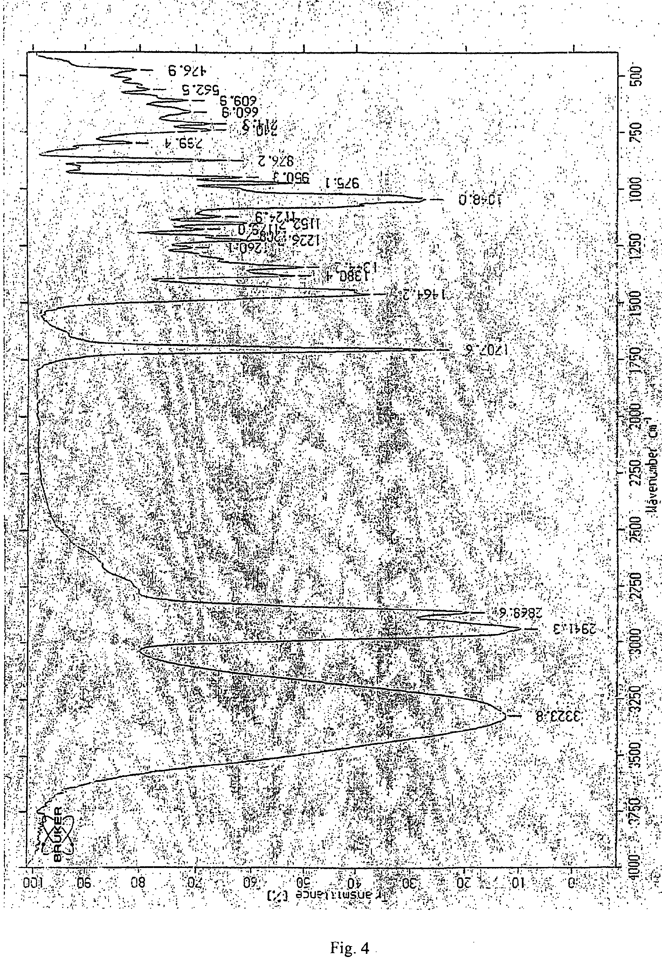

Fig.4 represents YC-1's infrared (ir) spectra.

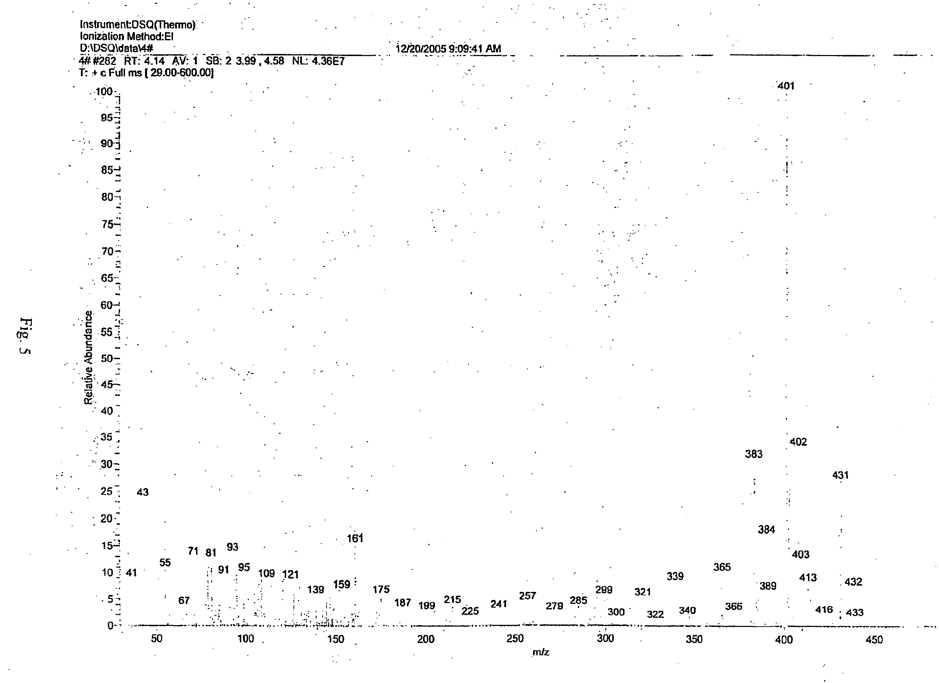

Fig.5 represents YC-1's masspectrometry (ms).

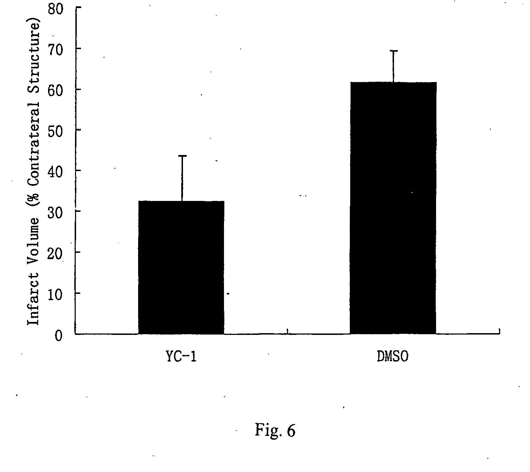

Fig.6 indicates YC-1 can reduce hemisphere infarct volume in permanent middle cerebral artery occlusion (MCAO).
Fig.7 represents YC-1's protection on rats with permanent middle cerebral artery occlusion (MCAO).
Fig.8 represents YC-1's effect on neurological function score of rabbit spinal cord ischemia model.
Fig.9 represents YC-1's effect on behavior of rabbit spinal cord ischemia model.
Fig.10 indicates YC-1 can increase the number of spinal cord anterior horn neurons after rabbit spinal cord ischemia.

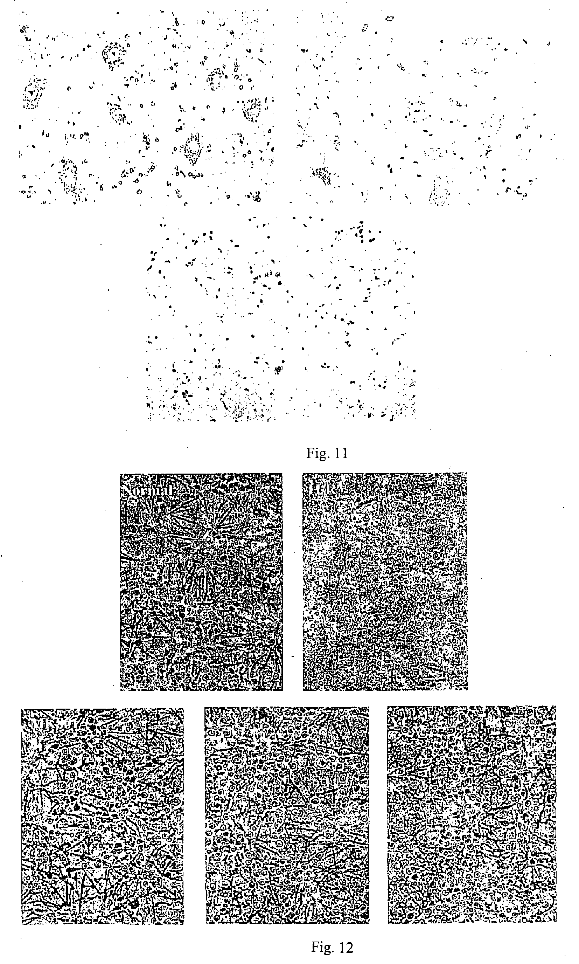

Fig.11 is a HE dye result indicating YC-1's effect on spinal cord anterior horn neurons after rabbit spinal cord ischemia.
Fig.12 represents CGNs morphology revealed by phase contrast microscope after sequential treatment of YC-1 and H/R.

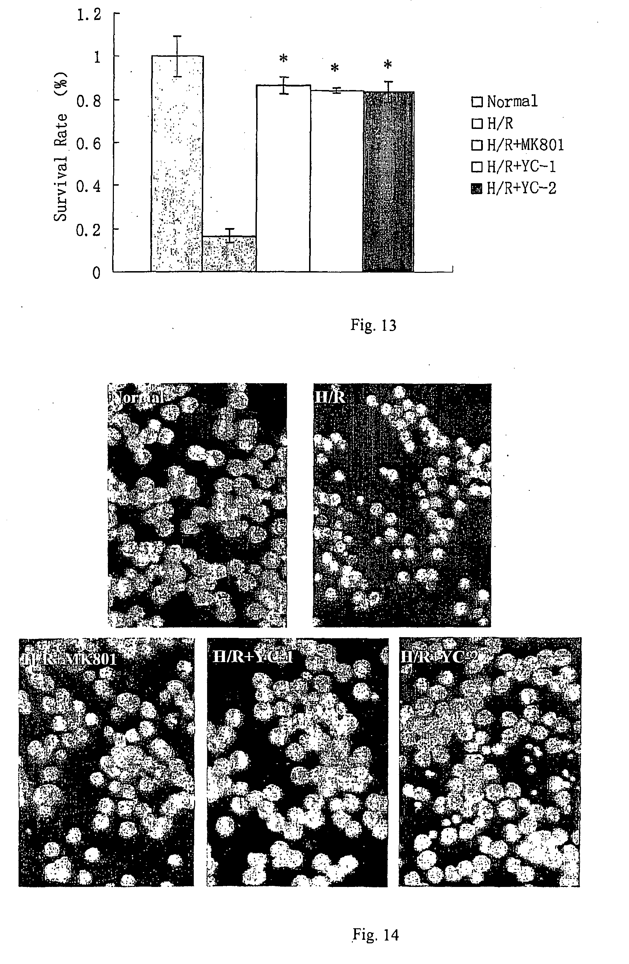

Fig.13 is a FDA dye result of viable cells indicating YC-1 can inhibit H/R induced CGNs apoptosis.
Fig.14 is the post-apoptosis Hoechst33258 nuclear morphology analysis indicating YC-1 can inhibit H/R induced CGNs apoptosis.

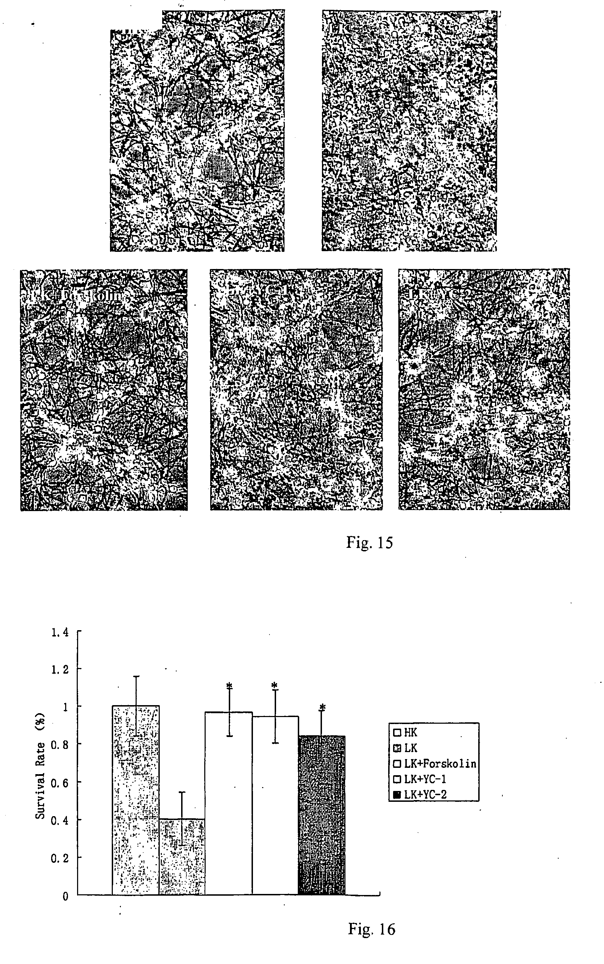

Fig.15 represents CGNs morphology revealed by phase contrast microscope after sequential treatment of YC-1 and LK.
Fig.16 is a FDA dye result of viable cells indicating YC-1 can inhibit LK induced CGNs apoptosis.

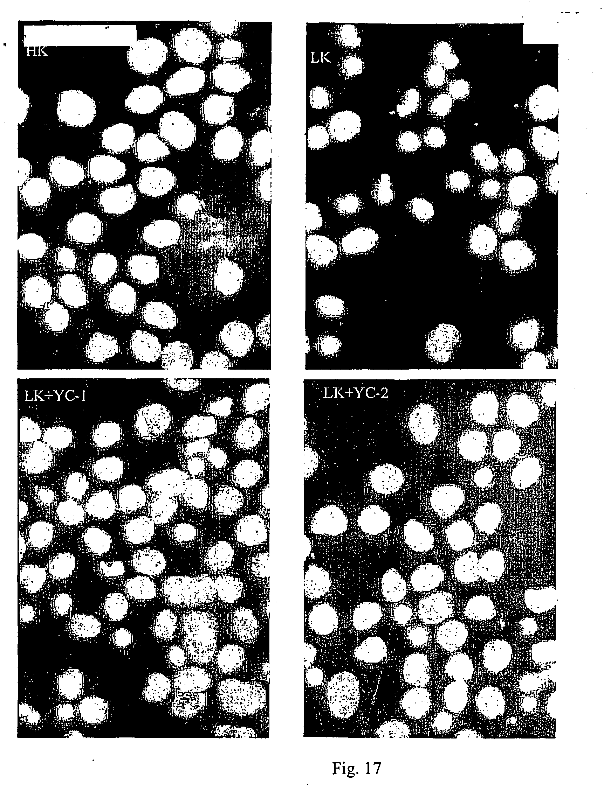

Fig.17 is the post-apoptosis Hoechst33258 nuclear morphology analysis indicating YC-1 can inhibit LK induced CGNs apoptosis.

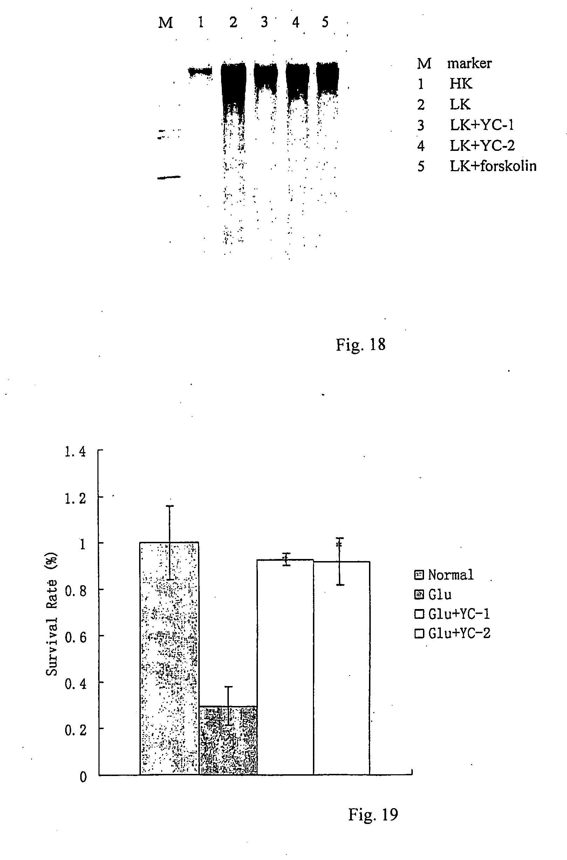

Fig.18 is the agarose gel electrophoresis analysis indicating YC-1 can reduce DNA fragments produced from LK induced CGNs apoptosis.
Fig.19 is a FDA dye result of viable cells indicating YC-1 can inhibit Glu induced CGNs apoptosis.

### EXAMPLES

### 1. Synthesis of marine steroid YC-1 (24-methylene-cholest-3β,5α,6β,19-tetrol)

Starting with cheap hyodeoxycholic acid, we synthesized YC-1 and some of it analogues through 14 steps of organic reactions; Then characterized the intermediates and target chemicals using spectrum technology.

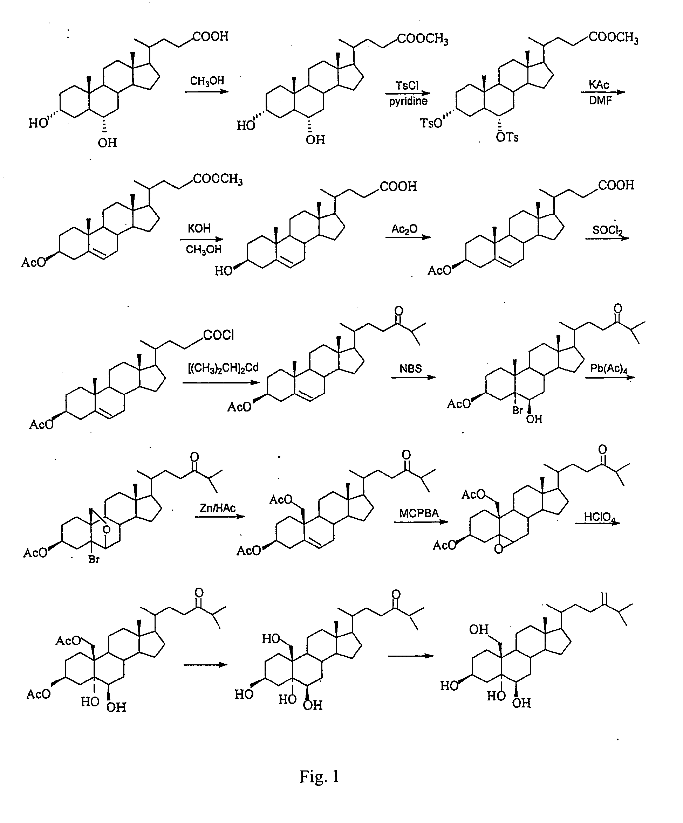

### 1.1 Synthetic route of YC-1 (24-methylene-cholest-3β,5α,6β, 19-tetrol), see Fig.1.

### 1.2 Optimization of YC-1's synthetic conditions

### 1.2.1 Synthesis of 3-acetoxy-cholest-5-Br-6-OH-24-ketone

It was synthesized through reaction of 3-acetoxy-cholest-5-ene-24-ketone and N-bromosuccinimide. The yield was low and there were multiple isomers in the product. The reaction was optimized by trying different conditions like solvents, temperature etc. Then tried different purification methods like recrystallization, solvent precipitation, chromatography etc.

### 1.2.2 Synthesis of 3-acetoxy-cholest-5-Br-6,19-epoxy-24-ketone.

It was synthesized by remote oxidative ring-closure of 6-OH in 3-acetoxy-cholest-5-Br-6-OH-24-ketone. Lead tetraacetate and diacetyl iodobenzene were used for the oxidation under uv light and in ultrasonic wave respectively and also optimized the reaction condition.

### 1.2.3 Synthesis of 3,19-diacetoxy-5,6-epoxy-24-ketone

It was done by oxidation of 5,6 double bond in 3,19-diacetoxy-5ene-24-ketone. Oxidation products got with different oxidant have different cis-trans ratio and yield. Totally there oxidants were used for the oxidation: m-chloroperoxybenzoic acid, hydrogen peroxide/formic acid system, and potassium permanganate/copper sulfate system. Then optimized the reaction condition.

### 1.3 Characterization of target chemical YC-1 and the intermediates.

YC-1 and the intermediates were characterized data like IR, 1H NMR, 13C NMR, MS and EA etc got from techniques like IR, NMR, MS and EA etc. Among whichYC-1's spectral data is:
Melting point: 223-225°C
Elemental analysis: C28H4804
Measured value: C, 75.20; H, 10.36
Theoretical value: C, 74.95; H, 10.78
**MS :** [M-H₂O]⁺=432,
**IR :** 3416 , 3028 , 2936 , 2868 , 1642, 1464 , 1377 , 1057

### ¹HNMR(DMSO)

5.25(1H,d,6β-OH), 4.71(1H,d,*J*=1.5Hz,28-CH), 4.65(1H,d,*J*=1.5Hz,28-CH), 4.51(1H,d, 19-OH), 4.16(1H,d,3-CH), 4.01(1H,d,19-CH), 3.84(1H,m,3-CH), 3.64(1H,s,5-OH), 3.23 (1H,m,6-CH), 2.25(1H,m,25-CH), 1.13(6H,d,26-CH₃,27-CH₃), 1.01(3H,s, 19 -CH₃), 0.87(3H,d, 21-CH₃), 0.67(3H,s,18-CH₃).

### ¹³CNMR(DMSO)

156.6(C), 107.3(CH₂), 75.4(C), 74.7(CH), 66.7(CH), 63.0(CH₂), 57.4(CH), 56.7(CH), 45.9(CH), 43.5(C), 43.3(CH), 42.8(C), 41.3(CH₂), 40.9(CH), 37.4(CH₂), 36.1(CH), 33.9(CH₂), 31.8(CH₂), 31.4(CH₂), 30.5(CH₂), 28.7(CH₂), 27.9(CH₂), 24.70(CH₂), 22.64(CH₃),22.57(CH₃), 19.4(CH₃), 13.1(CH₃) .

### 1.4 Design and synthesis of YC-1's analogues.

(1) 3-hydroxycholest series analogues
(2) 3,6-dihydroxycholest series analogues
(3) 3,5,6-trihydroxycholest series analogues
(4) 3,19-dihydroxycholest series analogues
(5) 3,5,6,19-tetrahydroxycholest series analogues
(6) 3-hydroxycholest dimer
(7) 3,5,6,19-tetrahydroxycholest dimmer
(8) The side chain at position 17 of chemicals in (1)-(7) can be: 24-carbonyl, 24-methylene, 24-hydroxy or 24-hydrazone etc. Then characterized them using techniques like IR, NMR, MS and EA etc.

### 1.5 Study of steroid structure modification

YC-1 has poor water solubility. To improve its solubility, the 3-hydroxy group in 3-hydroxy-cholest-5-ene-24-ketone went through hydrophilic modification.

### 1.5.1 Synthesis of cholest-5-ene-24-ketone-3-hydroxy-sulfonic ester

### 1.5.2 Synthetic study of PEG modified 3-hydroxy-cholest-5-ene-24-ketone

### 2. YC-1 and its analogues' effect on MCAO induced rat cerebral ischemia model

### Method: preparation of rat middle cerebral artery occlusion (MCAO) model

25 healthy male SD rats were divided into 3 groups randomly:
YC-1 group (n=11): 10min, 6h, 24h and 7d after occluding the middle cerebral artery, 12mg/Kg YC-1 was continuously injected intraperitoneally.
DMSO group (n=11): injected same volume of DMSO (1ml/Kg) in the same way.
Sham group (n=3): just very short intraluminal thread was inserted and didn't occlude the MCA.

The following method was a modification of Zea Longa's method (Longa EZ, Weinstein PR, Carlson S, Cummins R. Reversible middle cerebral artery occlusion without craniectomy in rats. Stroke, 1989, 20: 84-91).
(1) The rats drinked freely but fasted one night before the operation.
(2) 10min before anesthesia, intraperitoneally (ip) injected atropine sulfate (0.5mg/Kg).
(3) Narcotized the rat with ip injected 10% chloral hydrate, 3.5ml anesthetic per Kg rat; The areflexia of hindlimb retraction and corneal were used as anesthesia index.
(4) Supinely fixed the rat after anesthesia, cut the cervical skin and subcutaneous tissue, separated left cervical skin from digastric, sternocleidomastoid and omohyoid, exposed the common carotid artery (CCA) bifurcation, including the internal and external carotid artery (ICA, ECA) extended from CCA bifurcation in the triangle formed by the three muscles.
(5) Ligated the CCA and ECA at the beginning of ascending pharyngeal artery as follows: prepared a thread at the proximal end of ICA, incised CCA at the bifurcation, inserted a 5cm nylon intraluminal thread to ICA's proximal end, stopped when inserted about 17-18mm and felt resistance. At this moment the nylon thread entered ICA and anterior cerebral artery, blocked all blood sources for MCA, and timed immediately when blocking the blood flow.
(6) Fastened the prepared thread at proximal ICA, trimmed the redundant thread, disinfected with iodine tincture, sutured the skin and returned the rat back to cage.
(7) Rectum temperature was measured using Meikangti electronic temperature measure instrument the whole process and it was strictly controlled at 36.5-37.5°C.
(8) Except using shorter intraluminal thread (10mm) which didn't reach MCA, all other steps for the Sham group were the same as above.

**Result:** By visual inspection, the brain tissue in the Sham group had normal color and morphology with no pallor and swelling; Those in the DMSO group showed obvious pallor and swelling and no obvious pallor and swelling in the YC-1 group. TTC dye showed that YC-1 group had smaller infarct volume than DMSO group (the infarct size was 32±11% and 62±8% respectively) while no ischemia was observed in the Sham group.

### 3. YC-1's effect on abdominal aorta clipping induced rabbit spinal cord ischemia model.

### Method: Preparation of spinal cord ischemia model.

24 male new Zealand white rabbits were divided evenly into 3 groups (n=8): YC-1 group: 30min before spinal cord ischemia, 8mg/Kg YC-1 was intravenous injected through the rabbit's ear.
DMSO group: injected same volume of DMSO (1ml/Kg) in the same way.
Sham group: just exposed the abdominal aorta but didn't clip it.

Referring to Johnson etc, the model was prepared as follows:
(1) The rabbits drinked freely but fasted one night before the operation.
(2) Anesthetized the rabbit by ear intravenous injected 3% sodium pentobarbital (30mg/Kg). On the other side of the ear set a vein-detaining needle (24G) for medicine and other fluid infusion.
(3) Set an artery-detaining needle (24G) in the right ear artery for monitoring the proximal arterial pressure and blood sampling during the operation.
(4) Set an artery-detaining needle (24G) in a femoral artery for monitoring the distal arterial pressure during the operation.
(5) Supinely fixed the rabbit, incised at the median abdomen, exposed the abdominal aorta.
(6) Injected heparin (150U/Kg) through the vein-detaining needle, then clipped the abdominal aorta at the 0.5-1.0cm spot below origin of the left renal artery to cause spinal cord ischemia.
(7) Once the abdominal aorta was blocked, the distal arterial pressure decreased immediately and the femoral arterial pulsation disappeared.
(8) Removed the clip 20min after ischemia and sutured the abdomen.
(9) Then intramuscularly injected 40,000U gentamicin, returned the rabbit back to cage and observed for 48h.
(10) The proximal arterial pressure, distal arterial pressure and heart rate (Spacelab, US) were continuously monitored during the operation. Rectum temperature was maintained at 38.5±0.5°C using heating pad and roasting lamp.
(11) Blood gas (AVL-2, Switzerland) and glucose (One Touch II, USA) were monitored by ear artery blood sampling 10min before ischemia, 10min after ischemia and 10min after reperfusion.

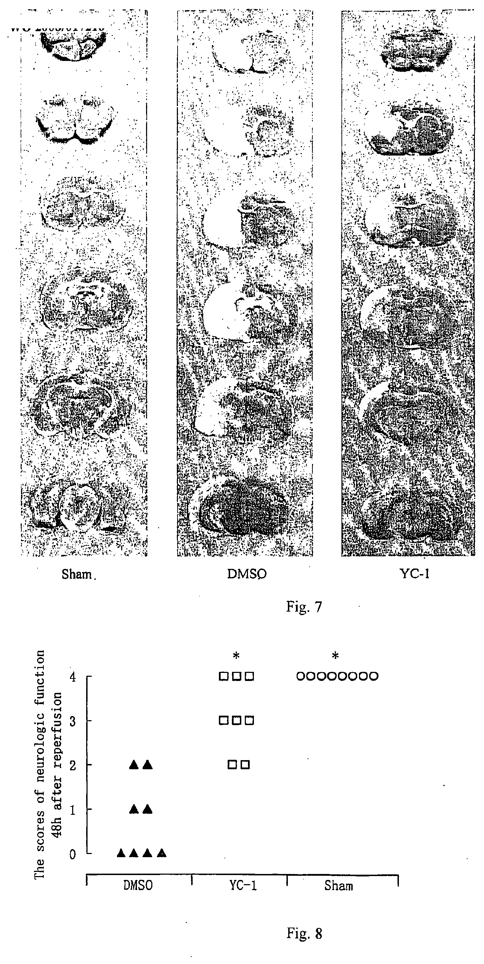

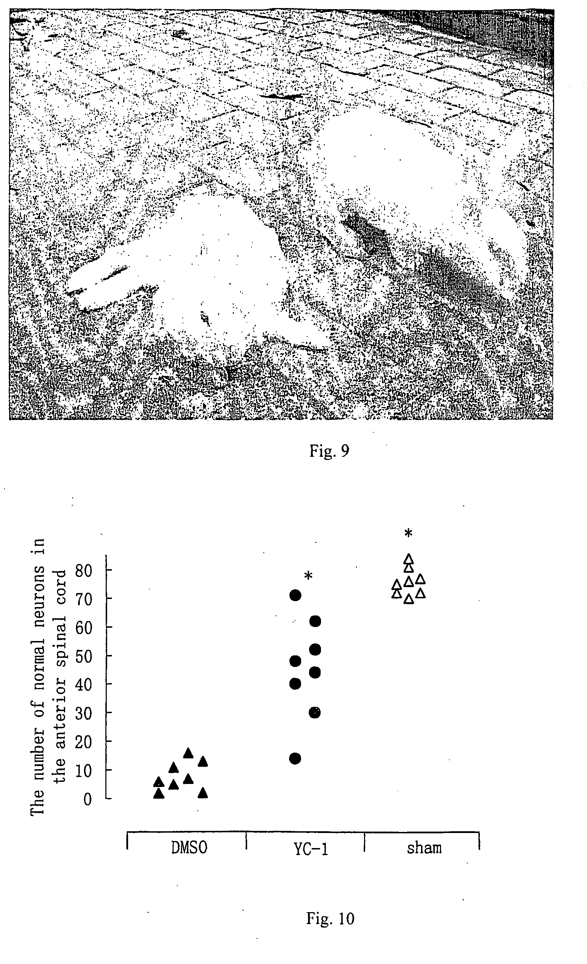

**Result:** See Fig.8 for neurological function score of animals from each group. Hindlimb nerve in the sham group functioned normally (4points) during the whole observation period. None rabbit in DMSO group could stand. Six rabbits in the CPA (YC-1)group could stand and the other two rabbits' hindlimbs also showed obvious movement. At each observation time point, neurological function score in YC-1 and sham groups were obviously higher than that in DMSO group (p<0.05). Histopathological examination showed that the lumbar spinal cord in DMSO group was severely injured manifesting as reduction of motor neurons, disappearance of nissl bodies and nucleus and vacuolar degeneration, while lumbar spinal cord injury in YC-1 group was obviously relieved, see Fig.9. Comparing to DMSO group, number of normal spinal cord anterior horn neurons in CPA(YC-1) and sham groups increased evidently (p<0.01), as shown in Fig.10.

### 4.YC-1 and its analogues' effect on hypoxia induced in vitro cultured cerebellar granule neurons death.

### Method:

### 1) Culture of rat cerebellar granule neurons (CGNs)

According to Yan, etc^{[34]}, prepared the neurons as follows:
(1) Took a clean 7-8d SD rat weighing about 15-20g, separated the cerebella under sterile condition.
(2) Got rid of the meninges and vessels in Kreb's anatomy solution ( Ca²⁺ and Mg²⁺ free ).
(3) Sheared the cerebella into pieces of 1mm³ with ophthalmic scissors.
(4) Digested in 0.25g/L trypsin solution for 15min at 37°C.
(5) Stopped the digestion with solution containing 0.5g/L trypsin inhibitor and 0.05g/L Dnase I, pipetted up and down to get single cell suspension.
(6) Centrifuged at 200g for 5min, rinsed the precipitation once with rinse solution.
(7) Span down again at 200g for 5min, discarded the supernatant.
(8) Diluted the cell pellet to cell density of 1.5-1.8 × 10⁶ cells/ml with BME medium containing 10% (v/v) FBA and 25mM KCl.
(9) Inoculated them into cell culture dishes pre-coated with polylysine and incubated at 37°C incubator with 5% CO₂.
(10) Twenty-four hours after inoculation, added 10 µmol/L Ara-C to inhibit non-neuron cells' growth and proliferation and to make the purity of CGNs above 95%.
(11) Added glucose to 5mM on day7 incubation to supplement energy needed by cell metabolism.
(12) Did the following experiment on day8.

### 2). Establishment of CGNs hypoxia/reoxygenation (H/R) injury model

Referred to Seko (Seko Y, Tobe K, Ueki K, et al. Hypoxia and hypoxia/reoxygenation activate Raf-1, mitogen-activated protein kinase kinase, mitogen-activated protein kinases, and S6 kinase in cultured rat cardiac myocytes. Circ Res. 1996 Jan;78(1):82-90) etc for the establishment method:
(1) On day8, put the treated CGNs in airtight box with oxygen consumption reagent and indicator and incubated at 37°C for 3h.
(2) Transferred the cells to 37°C incubator with 5% CO₂ for reoxygenation treatment.

### 3). Cell morphological observation and neuron viability measurement

The following were done referring to Yan (Yan G M, Irwin R P, Lin S Z, et al. Diphenylhydantoin induces apoptotic cell death of cultured rat cerebellar granule neurons. J Pharmacol Exp Ther, 1995, 274(2): 983-8) et al.:
(1) CGNs were cultured in 35mm dishes, on day8, removed the medium after various factors treatment, rinsed the cells twice with 4°C PBS containing Ca²⁺ and Mg²⁺.
(2) Fixed the cells with 40g/L paraformaldehyde (4°C, dissolved in PBS) for 10min.
(3) Removed the fixation solution, rinsed the cells twice with distilled water, air dried at 4°C.
(4) Observed the cell morphological change and randomly photographed under inverted fluorescence microscope (Olympus).

### Result:

### 1) Result got under inverted fluorescence microscope:

(1) Before hypoxia, CGNs cell bodies were plump and lucent; Cell processes and the network formed by them were tight, clear and intact.
(2) After 3h hypoxia and 24h reoxygenation (H/R) treatment, the CGNs cell body dwindled; The structure became unclear; The processes and network showed disorder and fracture.
(3) First treated the cells with 10µM MK801 or 12µM YC-1 or 12µM YC-2 for 3h, then did the H/R treatment and observation. Most CGNs cell body kept intact, plump and lucent; The processes and network were tight, clear and intact.

### 2) FDA dye analysis:

(1) After H/R treatment, neuronal viability decreased obviously (16.56±3.21%).
(2) After treated by 10µM MK801 or 12µM YC-1 or 12µM YC-2, neuronal viability reached 86.73±3.21%, 84.29±1.26% and 83.54±4.78% respectively.

### 3) Hoechst33258 dye analysis:

(1) Normal CGNs had intact nucleus and the chromatin distributed evenly.
(2) After H/R treatment, observed CGNs nucleus and chromatin pyknosis together with apoptotic bodies.
(3) First treated the cells with 12µM YC-1 YC-2, then did the H/R treatment and observation. Most CGNs showed normal nuclear morphology and even chromatin distribution.

### 5. YC-1 and its analogues' effect on repolarization induced in vitro cultured cerebellar granule neurons death.

### Method:

The methods for rat CGNs cell culture, cellular morphologic observation and neuronal viability measurement were the same as above. Then check the in vitro cultured cerebellar granule neurons death through agarose gel electrophoresis according to reference^{[34]}:
(1) inoculated CGNs in 35mm² dishes.
(2) On day8, 24h after adding various medicines, removed the medium, rinsed the cells twice with PBS.
(3) Span down the collected cells with a low temperature high speed centrifuge (Biofuge22R, Heraeus, Germany) at 5000rpm, 4°C for 5min.
(4) Resuspended the cell pellet with 600µl buffer containing 10mmol/L Tris-HCl, 10 mmol/L EDTA and 2g/L Triton X-100 (pH 7.5) and incubated for 15min.
(5) Span down at 12000rpm, 4°C for 10min.
(6) Extracted the supernatant once with equal volume of phenol, span down.
(7) Extracted the supernatant once with equal volume of phenol-chloroform (1:1), span down.
(8) Took the supernatant, added 300mmol/L Sodium Acetate and equal volume of isopropanol, precipitated for overnight.
(9) Span down the pellet, rinsed 2-3 times with 70% ethanol.
(10) After drying, added some TE (Tris 10mmol.L⁻¹, EDTA 1.0 mmol.L⁻¹, pH 7.4) and 0.6g.L⁻¹ 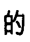 RNase A and incubated at 37°C for 30min.
(11) Analyzed the samples with 20g/L agarose gel electrophoresis for 1h.
(12) Dyed with ethidium bromide and photographed under UV light.

### Result:

### (1) Rat CGNs in vitro cultured in serum free BME medium containing 25mM KCl for 8 days showed intact cell bodies and processes.

(1) Transferred above cells to serum free medium with 5mM KCl and incubated for another 24h, the cell dodies dwindled and the nucleus fractured; FDA fluorescence staining indicated only 40.2±14.04% CGNs survived.
(2) If 12µM YC-1 or YC-2 were added 3h before and the same time changing to low potassium medium, then cultured the cells in the low potassium medium for another 24h, most CGNs kept intact cell bodies and processes; FDA staining indicated 94.3± 14.02% and 84.2±13.34% CGNs survived respectively.
(4) These results showed marine steroid YC-1 and YC-2 obviously improved rat CGNs' viability in low potassium medium.

### 6. YC-1 and its analogues' effect on glutamic acid (Glu) induced in vitro cultured cerebellar granule neurons death.

### Methods:

(1) Isolatation and primary culture of rat CGNs.
(2) Morphology observation of neuron cell body and processes under phase contrast microscopy.
(3) Analysis of neuronal metabolic activity and viability using fluorescein diacetate (FDA) staining.

### Result:

(1) Took rat CGNs in vitro cultured for 8 days, added 150µM Glu, incubated for 24h and observed under phase contrast microscopy. The neuronal cell bodies dwindled obviously, the nucleus showed pyknosis and the processes fractured or disappeared.
(2) If 12µM YC-1 or YC-2 were added 1h before adding Glu, then observed 24h later. The result showed that both YC-1 and YC-2 could obviously inhibited Glu induced CGNs apoptosis.
(3) CGNs' Viability analysis using FDA staining showed that 12µM YC-1 and YC-2 clearly improved CGNs' viability (respectively 92.6±2.59% and 91.6±10.11%) comparing to the Glu control group (29.5±8.23%).

## Claims

1. An application of marine steroid chemicals in preparing medicines for neuron damage.

2. An application according to claim1, the marine steroid chemical is 24-methylene-cholest-3β,5α,6β,19-tetrol.
